Europäisches Patentamt

European Patent Office

Office européen des brevets

(11) Publication number: **0 455 433 A1**

# (12) EUROPEAN PATENT APPLICATION

(21) Application number: **91303803.0**

(22) Date of filing: **26.04.91**

(51) Int. Cl.⁵: **A61M 16/18, F16K 35/00**

(30) Priority: **01.05.90 GB 9009730**

(43) Date of publication of application:
**06.11.91 Bulletin 91/45**

(84) Designated Contracting States:
**DE ES FR GB IT NL**

(71) Applicant: **The BOC Group plc**
**Chertsey Road**
**Windlesham Surrey GU20 6HJ (GB)**

(72) Inventor: **Whitaker, Keith**
**3 Hall Court, Sutton-in-Craven**
**Keighley, West Yorkshire (GB)**

(74) Representative: **Belcher, Simon James et al**
**Urquhart-Dykes & Lord Tower House Merrion**
**Way**
**Leeds LS2 8PA (GB)**

(54) **Fluid delivery system.**

(57)   A system for delivering fluid to a vessel in dispensing apparatus for the fluid, such as anaesthetic dispensing apparatus. which includes an opening (12) for receiving a fluid delivery conduit (14, 16), means (26, 50) for clamping the delivery conduit in position in the opening, a valve (28,46) by which access of fluid to the interior of the vessel can be controlled, a cam (38) which is movable by the clamping means from a first position in which it prevents opening of the valve to a second position in which the valve is openable, and a restrictor (32) which is movable by the fluid delivery conduit when inserted into the opening, from a first position in which it impedes movement of the cam and thereby prevents opening of the valve, to a second position in which it does not impede the movement of the cam and in which the valve can be opened.

The operative connections between the components of the delivery system ensures that the valve can only be opened when the fluid delivery conduit is received and clamped in the opening in the fluid reception conduit.

FIG. I

The present invention relates to fluid delivery systems and in particular to devices for filling anaesthetic vaporisers with liquid anaesthetic agent.

Throughout this specification the term "anaesthetic" is intended to embrace anaesthetic and analgesic agents.

As explained in UK Patent No. 1193241, anaesthetic vaporisers are used with anaesthesia machines for mixing the vapour of a volatile liquid anaesthetic with a carrier gas such as air, oxygen, nitrous oxide or a combination thereof.

Usually, an anaesthetic vaporiser is dedicated for use with only one type of anaesthetic and UK Patent No. 1193241 and UK published patent application No. 2189472 each describe a filling system which substantially eliminates the possibility of an anaesthetic vaporiser being filled with the wrong anaesthetic agent.

In these known systems, coding for drug type is achieved by means of a bottle adaptor which at one end has a cap for screwing to a bottle containing liquid anaesthetic agent and at its opposite end is formed with an outlet termination for location in an opening in a filler block forming part of the anaesthetic vaporiser. The cap has slots which correspond to and co-operate with lugs formed on a free spinning keyed bottle collar placed on the neck of the bottle. The outlet termination is also keyed, for example a slot is positioned and/or dimensioned to mate with a locating tongue or peg on the filler block so that only an outlet termination of the correct shape can enter the opening in the filler block.

In these known systems, when it is desired to fill an anaesthetic vaporiers with liquid anaesthetic agent, the outlet termination of the appropriate bottle adaptor is located in the opening in the filler block. A clamp lever is then tightened and the bottle containing liquid anaesthetic agent attached to the cap of the bottle adaptor is lifted above the level of the opening. Next a valve is opened allowing liquid anaesthetic agent to enter the vaporiser via the filler block from the bottle.

A disadvantage of this known system is that the valve can be opened before the outlet termination is fully inserted and clamped in the opending of the filler block. If the valve is opened with no outlet termination inserted any drug which is present in the vaporiser sump will leak out.

As well as being wasteful this could create a hazardous situation.

The present invention provides provides a fluid delivery system comprising:

(a) a fluid reception conduit for connection to a vessel to be filled with fluid, the receipt conduit having an opening for receiving a fluid delivery conduit through which fluid is supplied to the vessel;

(b) means for clamping the delivery conduit in position in the opening;

(c) a valve by which access of fluid to the interior of the vessel can be controlled;

(d) a cam which is movable by the clamping means from a first position in which it prevents opening of the valve to a second position in which the valve is openable; and

(e) a restrictor which is movable by the fluid delivery conduit when inserted into the opening, from a first position in which it impedes movement of the cam and thereby prevents opening of the valve, to a second position in which it does not impede the movement of the cam and in which the valve can be opened.

The fluid delivery system of the present invention has the advantage that the valve by which access of fluid to the interior of the vessel is controlled can only be opened when the fluid delivery conduit is received and clamped into the opening in the fluid reception conduit. As a result, it is not possible for fluid contained within the vessel to leak out through the open valve in the absence of the fluid delivery conduit. As a result, wastage of fluid is avoided, as is the potentially hazardous situation which might arise in the event that the leaking fluid has damaging properties.

Preferably, the link between the cam and the valve has the form of a peg, which moves in a slot. Preferably, the peg is provided on the cam, and it moves in a slot provided on a component of the valve. Preferably, the slot has a first portion in which, when the peg is located in it, the valve is prevented from opening. The slot preferably also has a second portion in which, when the peg is located in it, the valve can be opened. In this arrangement, the said first position of the cam is that in which the peg is located in the first portion of the slot, and the said second position of the cam is that in which the peg is located in the second portion of the slot. When the valve moves between its open and closed positions pivotally, the second portion of the slot will generally extend in a radial direction relative to the point about which the valve moves, and the second portion of the slot will generally extend around and arc relative to that pivot point.

Preferably, movement is transmitted between the clamping means and the cam by means of cooperating teeth, preferably in the manner of components of a gear system. Preferably, one of the clamping means and the cam bears a pair of teeth, into which a tooth on the other of the clamping means and the cam can be received. In this way, movement in two opposite directions can be transmitted between the clamping means and the cam.

Preferably, movement of the cam or the restrictor or both involves pivotal movement. Preferably, movement of the valve between its open and closed position involves pivotal movement.

Preferably, the restrictor is biased resiliently towards its first position. This has the advantage that, in the absence of the fluid delivery conduit from the opening in the fluid reception conduit, the valve will not be openable.

An embodiment of the invention will now be described by way of example, reference being made to the Figures of the accompanying diagrammatic drawings in which:-

Figure 1 is a perspective view of a known filler block forming part of an anaesthetic vaporiser and the outlet termination of a bottle adaptor;

Figure 2 is a perspective view similar to Figure 1 but showing the anaesthetic vaporiser being filled with a liquid anaesthetic agent;

Figure 3 is a plan view of a detail showing the relationship between a cam and a cam restrictor forming part of the filler block modified to prevent opening of a valve lever before the outlet termination of the bottle adaptor is fully inserted and clamped in the opening of the filler block;

Figures 4 and 5 are cross-sections on line A-A of Figure 3 showing the cam restrictor in two alternative positions;

Figure 6 is a perspective view of a detail including a clamp lever actuator, a cam and a valve lever in its open position forming part of the filler block of Figure 3;

Figure 7 is a plan view of the detail of Figure 6;

Figure 8 is a perspective view of the detail of Figure 6 but with the valve lever in its closed position; and

Figure 9 is a plan view of the detail of Figure 8.

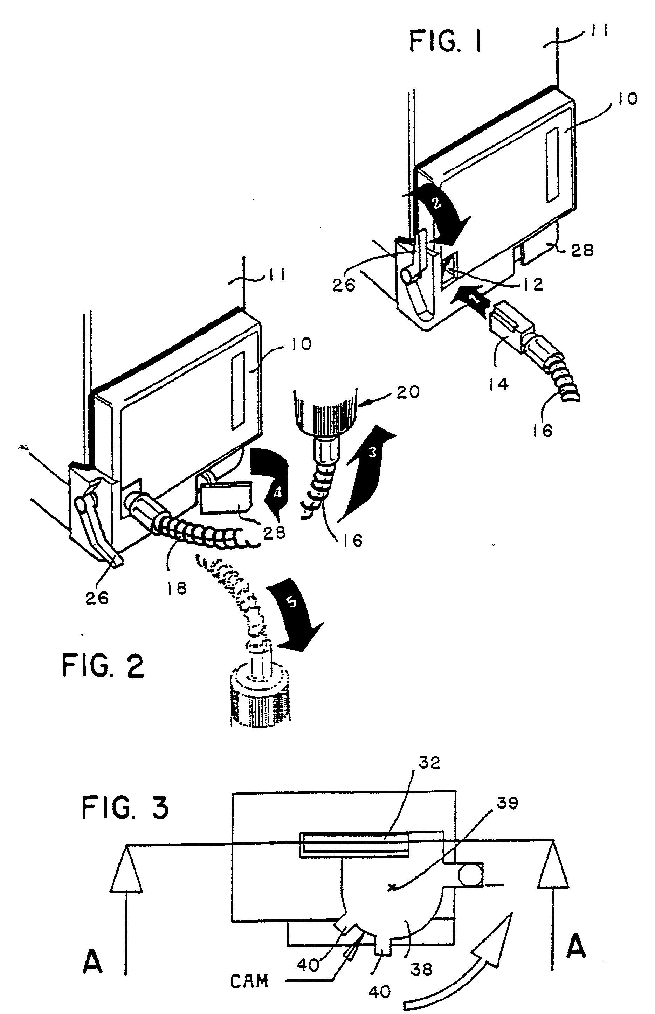

Referring first to Figures 1 and 2, a filler block 10 forming part of an anaesthetic vaporiser 11 includes an opening 12 which receives an outlet termination 14 forming part of a bottle adaptor 16. The bottle adaptor 16 includes a tube 18 connecting the outlet termination 14 to a cap connection 20. The cap connection 20 is screwed on to the threaded neck of a bottle (not shown) containing liquid anaesthetic agent and slots in a cap forming part of the cap connection co-operate with lugs on a coded collar (not shown) arranged around the neck of the bottle.

Usually, the outlet termination 14 is keyed, for example, a slot is positioned and/or dimensioned to mate with a locating tongue or peg on the filler block 10 so that only an outlet termination 14 of the correct shape can enter the opening 12.

When it is desired to fill the vaporiser 11 with liquid anaesthetic agent, the outlet termination 14 is located in the opening 12 and a clamp lever 26 is tightened by rotation from the position shown in Figure 1 to the position shown in Figure 2. The bottle is lifted as illustrated in Figure 2 and a valve lever 28 is opened allowing liquid anaesthetic agent to enter the vaporiser via the filler block 10 from the bottle.

The above described bottle adaptor and method of filling an anaesthetic vaporiser are well known.

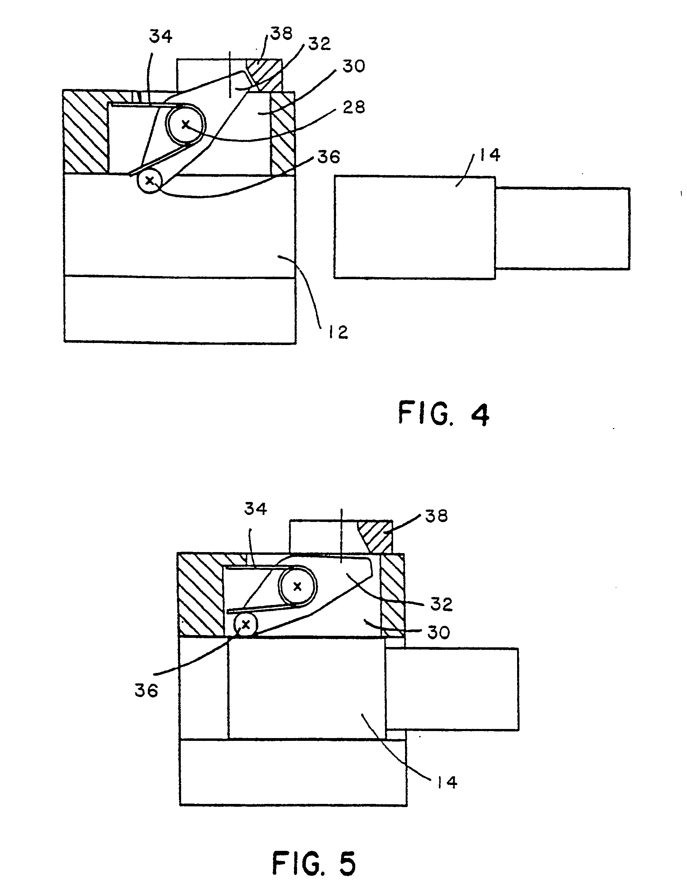

Referring now to Figure 3 to 5, mounted for rotation around a horizontal axis 28 in a recess 30 formed in the filler block 10 adjacent the opening 12 is a cam restrictor 32. As shown in Figure 4, a spring 34 reacts against a lug 36 formed on the restrictor 32 so that a portion including the lug 36 at one end of the restrictor 32 extends into the opening 12. At its opposite end a portion extends into the opening 12. At its opposite end a portion of the restrictor 32 extends into the path of a cam 38 mounted for rotary movement about a vertical axis 39.

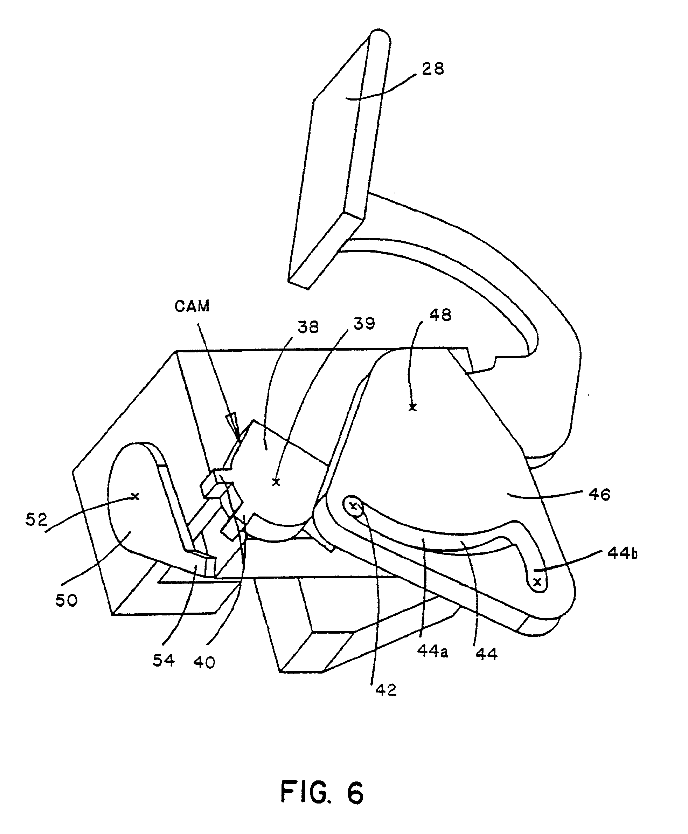

Referring also to Figures 6 to 9, the cam 38 is formed with peripheral teeth 40 and a peg 42. The peg 42 is located in a slot 44 formed in a plate 46 attached for movement with valve lever 28. The slot 44 has two portions namely a portion 44A and a portion 44B approximately at right angles to the portion 44A. The valve lever 28 and hence the plate 46 rotate about a vertical axis 48 and the slot portion 44A throughout its length is radially equidistant from the axis 48.

Mounted adjacent the cam 38 is a clamp lever actuator 50 mounted on a horizontal axis 52. The clamp lever actuator 50 is arranged to move with the clamp lever 26. The clamp lever actuator 50 includes an arm 54 which when the clamp lever 26 and hence the clamp lever actuator 50 are turned about the axis 52, engages the teeth 40 to rotate the cam 38 about the axis 39.

Prior to insertion of the outlet termination 14 into the opening 12, the restrictor 32 assumes the position illustrated in Figure 4 with its lower (as shown) end protruding into the opening 12 and its upper (as shown) end extending into the path of the cam 38 thereby preventing rotary movement of said cam 38.

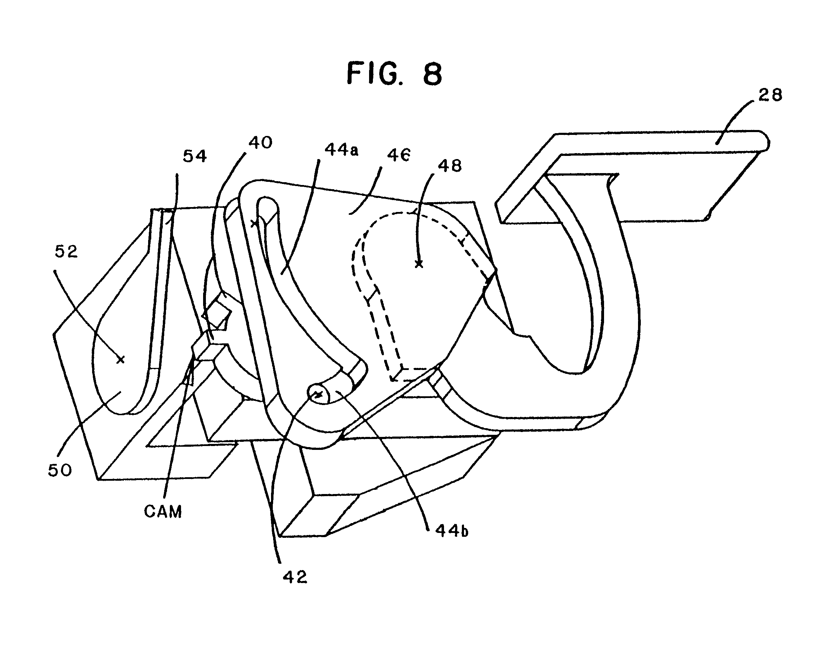

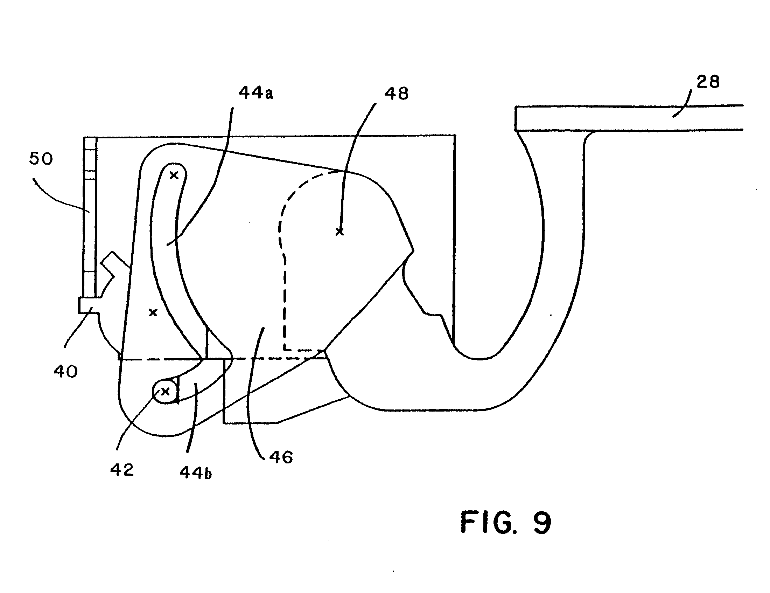

In this position the peg 42 will occupy a position in the slot portion 44B and the clamp lever actuator 50 will assume the position shown in Figures 8 and 9. It will be evident, in this position of the peg 42 in the portion 44B, that it is impossible to move the valve lever about the axis 48.

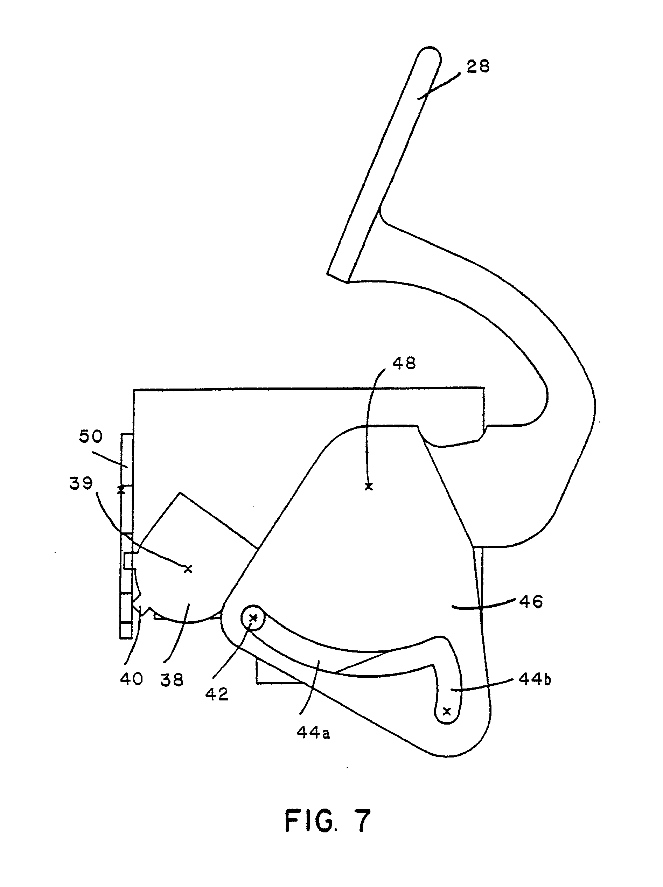

In use, when it is desired to fill the vaporiser 11 with liquid anaesthetic agent, the outlet termination 14 is inserted into the opening 12 which causes the restrictor 32 to move clockwise (as shown) against the bias of spring 34 into the position shown in Figure 5. As shown in Figure 5, this causes the upper portion of the restrictor to move clear of the cam 38. Once the restrictor 32 is clear of the cam 38 it is then possible to rotate the clamp lever 26 and hence the clamp lever actuator 50 from the position shown in Figure 1 to the position shown in Figure 2 which will cause the arm 54 to engage the teeth 40 on the cam 38 thereby moving the cam anti-clockwise around the axis 39. This has the effect of moving the peg 42 from the slot portion 44B into the slot portion 44A. It is only then possible that the valve lever 28 can be moved to the open position shown in Figures 6 and 7.

It will be evident that in this position of the valve lever 28 it is impossible to move the clamp lever actuator 50 anticlockwise about axis 52 since the teeth 40 on the cam 38 will prevent such movement.

When it is desired to close the valve lever 28 and remove the outlet termination 14 from the opening 12 then the valve lever is returned to the position shown in Figures 8 and 9 which will cause the peg 42 to move to the junction between the slot portions 44A and 44B. The clamp lever 26 and clamp lever actuator 50 can then be moved anti-clockwise causing the arm 54 to engage the teeth 40 and move the cam clockwise and hence the peg 42 along the slot portion 44B to the position shown in Figures 8 and 9. The outlet termination 14 is then removed from the opening 12 in the filler block 10 and the anaesthetic vaporiser is then ready for use.

It will be evident that in the above described embodiment it is impossible to open the valve lever 28 before the outlet termination 14 is fully inserted into the opening 12 and clamped therein by means of the clamp lever 26. Furthermore, without the outlet termination 14 in place in the opening 12 it is impossible to operate the clamp lever 26 thus giving a complete fail-safe interlock off all three items namely the bottle adaptor, clamp lever and valve lever.

When the vaporiser is full of anaesthetic agent the interlock mechanism prevents the removal of the outlet termination 14 before the valve lever 28 is closed thereby safeguarding against spillage of anaesthetic agent.

Although reference has been made in the above described embodiment to an anaesthetic vaporiser, the fluid delivery system together with the interlock mechanism could be applied to the filling of other types of vessels in which it is important that a valve providing access to the interior of the vessel cannot be opened until the delivery means is clamped in position ready to deliver fluid.

## Claims

1.  A fluid delivery system comprising:
    (a) a fluid reception conduit (10) for connection to a vessel to be filled with fluid, the reception conduit having an opening (12) for receiving a fluid delivery conduit (14, 16) through which fluid is supplied to the vessel;
    (b) means (26, 50) for clamping the delivery conduit in position in the opening;
    (c) a valve (28,46) by which access of fluid to the interior of the vessel can be controlled;
    (d) a cam (38) which is movable by the clamping means from a first position in which it prevents opening of the valve to a second position in which the valve is openable; and
    (e) a restrictor (32) which is movable by the fluid delivery conduit when inserted into the opening, from a first position in which it impedes movement of the cam and thereby prevents opening of the valve, to a second position in which it does not impede the movement of the cam and in which the valve can be opened.

2.  A fluid delivery system as claimed in claim 1, which includes a link between the cam (38) and the valve (28, 46) in the form of a peg (42) provided on one of the cam and the valve, which moves in a slot (44) in the other of the cam and the valve.

3.  A fluid delivery system as claimed in claim 2, in which the slot (44) has:
    (a) a first portion (44A) in which, when the peg (42) is located in it, the valve (28, 46) is prevented from opening; and
    (b) a second portion (44B) in which, when the peg (42) is located in it, the valve can be opened.

4.  A fluid delivery system as claimed in any one of claims 1 to 3, in which movement is transmitted between the clamping means (26, 50) and the cam (38) by means of cooperating teeth (40, 54).

5.  A fluid delivery system as claimed in any one of claims 1 to 4, in which the movement of the cam (38) between its first and second positions involves pivotal movement.

6.  A fluid delivery system as claimed in any one of claims 1 to 5, in which the movement of the restrictor (32) between its first and second positions involves pivotal movement.

7.  A fluid delivery system as claimed in any one of claims 1 to 6, in which the movement of the valve (28, 46) between its open and closed positions involves pivotal movement.

8.  A fluid delivery system as claimed in any one of claims 1 to 7, in which the restrictor (32) is biassed towards its first position.

9.  A fluid delivery system as claimed in any one of claims 1 to 8, which forms part of an anaesthetic supply system.

FIG. 1

FIG. 2

FIG. 3

CAM

A        A

FIG. 4

FIG. 5

FIG. 6

FIG. 7

FIG. 8

FIG. 9

European Patent
Office

**EUROPEAN SEARCH REPORT**

Application Number

EP 91 30 3803

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 434 790 (OLESEN)<br><br>* abstract; figures 3-9 *<br>* column 5, line 55 - line 66 * | 1,2,5,7,9 | A61M16/18<br>F16K35/00 |
| Y | EP-A-38 768 (S.A. DES ETABLISSEMENTS STAUBLI)<br><br>* page 4, line 5 - page 5, line 18 *<br>* abstract; figures 1-5 * | 1,2,5,7,9 | |
| A | FR-A-2 570 609 (DRÄGERWERK AG)<br>* abstract; figures 2-4 *<br>* page 2, line 32 - page 3, line 30 * | 1 | |
| A | EP-A-295 671 (DRÄGERWERK AG)<br>* abstract; figures 2,3 * | 1 | |
| | | | TECHNICAL FIELDS SEARCHED (Int. Cl.5) |
| | | | A61M<br>F16K |

The present search report has been drawn up for all claims

| Place of search | Date of completion of the search | Examiner |
|---|---|---|
| THE HAGUE | 28 JULY 1991 | ZEINSTRA H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)